Europäisches Patentamt

European Patent Office

Office européen des brevets

(11) Publication number: **0 041 702**
**B1**

(12) **EUROPEAN PATENT SPECIFICATION**

(45) Date of publication of patent specification: **05.06.85**

(21) Application number: **81104318.1**

(22) Date of filing: **04.06.81**

(51) Int. Cl.⁴: **C 12 M 1/04**

(54) **Apparatus for culturing microorganisms by aeration.**

(30) Priority: **06.06.80 JP 75596/80**

(43) Date of publication of application:
**16.12.81 Bulletin 81/50**

(45) Publication of the grant of the patent:
**05.06.85 Bulletin 85/23**

(84) Designated Contracting States:
**DE GB**

(56) References cited:
**US-A-3 846 245**
**US-A-4 112 829**

**DERWENT JAPANESE PATENTS REPORT, vol. 5, no. 7, B page 9, 2nd April 1971, no. 12958s; CHEMICAL ABSTRACTS, vol. 93, no. 19, 10th November 1980, page 491, no. 184228e, Columbus Ohio (USA); V.I. GOROVOI: "State of and prospects for the development of the use of secondary material resources in the alcohol industry".**

(73) Proprietor: **Hitachi, Ltd.**
**5-1, Marunouchi 1-chome**
**Chiyoda-ku Tokyo 100 (JP)**

(72) Inventor: **Yamaguchi, Tetsuo**
**43-26, Nishinarusawacho-4-chome**
**Hitachi-shi (JP)**
Inventor: **Saito, Setsuo**
**26-3, Higashionumacho-2-chome**
**Hitachi-shi (JP)**
Inventor: **Shimizu, Norio**
**2374-24, Okubocho**
**Hitachi-shi (JP)**
Inventor: **Ueno, Masao**
**20-3, Ayukawacho-6-chome**
**Hitachi-shi (JP)**
Inventor: **Odawara, Yoji**
**21-6, Nishinarusawacho-4-chome**
**Hitachi-shi (JP)**

(74) Representative: **Ebbinghaus, Dieter et al**
**v. FÜNER, EBBINGHAUS, FINCK Patentanwälte**
**European Patent Attorneys Mariahilfplatz 2 & 3**
**D-8000 München 90 (DE)**

Courier Press, Leamington Spa, England.

## Description

The invention relates to an apparatus for culturing microorganisms by aeration, which comprises a fermentor for aerobically culturing microorganisms, an oxygen production unit for producing an oxygen-enriched gas, a carbon dioxide-removing unit for separating and removing carbon dioxide produced during fermentation from an effluent gas from the fermentor and recycling the effluent gas free from the carbon dioxide to the fermentor, the fermentor and the carbon dioxide-removing unit being connected to each other in a loop.

In culturing microorganisms by aeration, carbon dioxide gas is produced at the propagation of microorganisms. In the conventional culturing by air blowing, the concentration of microorganism cells is low in the culture medium, and carbon dioxide gas is produced at a low rate. Thus, the carbon dioxide gas can be removed together with a large volume of effluent air, and the influence of carbon dioxide gas upon the propagation of microorganisms is not so serious except for a special case. However, when microorganisms are cultured at an elevated concentration of microorganism cells in the culture medium with an oxygen-enriched gas, carbon dioxide is produced at a high rate. Furthermore, enormous energy is required for the preparation of oxygen-enriched gas, and thus oxygen utilization of oxygen-enriched gas to be led to the fermentor must be increased to a maximum. Consequently, a gas-liquid contactor with good efficiency is used to reduce the rate of oxygen-enriched gas to be led to the fermentor or the effluent gas from the fermentor is recycled. However, if the oxygen utilization is increased without removing carbon dioxide gas from the effluent gas, the carbon dioxide gas is more and more accumulated in the fermentor. Thus, the concentration of carbon dioxide in the culture medium is increased, directly giving an adverse effect upon the propagation of microorganisms. Furthermore, the oxygen partial pressure is so lowered that the oxygen supply becomes short.

Thus, in aerobically culturing microorganisms in a fermentor by aeration with an oxygen-enriched gas, it is desirable to increase the oxygen utilization of oxygen-enriched gas and to effectively remove the carbon dioxide gas produced during the culturing at the same time.

In the conventional apparatus for aerobically culturing microorganisms in a fermentor by aeration with an oxygen-enriched gas produced in an oxygen production unit, wherein a carbon dioxide-removing unit is provided in a system for recycling an effluent gas from the fermentor to the fermentor, it has been proposed to provide an oxygen production unit by water electrolysis, the oxygen gas generated therein being led to the fermentor, and to provide a carbon dioxide-removing unit for removing the carbon dioxide from the effluent gas by an aqueous caustic soda solution (Japanese Patent Publication No. 6392/ 71), where the oxygen production cost is high in the oxygen production unit by water electrolysis, and thus the apparatus is not used when a high rate of oxygen is required. Furthermore, no cooling medium of low temperature can be obtained in the apparatus. On the other hand, the carbon dioxide-removing unit by use of an aqueous caustic soda solution requires a large amount of caustic soda and water for removing a high rate of carbon dioxide produced during the culturing, and also a large amount of the resulting aqueous sodium carbonate solution must be treated. When sodium carbonate is recovered as a by-product, a concentrating unit is additionally required, making the entire operation of the apparatus complicated.

As described above, any practical apparatus for culturing microorganisms by aeration on a large scale with a satisfactory effect, which is based on the introduction of an oxygen-enriched gas produced in an oxygen production unit into a fermentor to aerobically culture microorganisms and provision of a carbon dioxide-removing unit in a system for recycling an effluent gas from the fermentor to the fermentor, has not been realized yet.

An object of the present invention is to provide an apparatus for efficiently culturing microorganisms by aeration with an oxygen-enriched gas on a large scale, which can increase utilization of oxygen introduced into a fermentor by a simple means, and can continuously and satisfactorily separate and remove carbon dioxide produced in the fermentor to prevent various troubles due to accumulated carbon dioxide.

According to the present invention the apparatus for culturing microorganisms by aeration is characterized in that the oxygen production unit is of cryogenic separation type and is connected to the carbon dioxide-removing unit at the inlet, and in that the carbon dioxide-recovering unit is of heat exchange type of directly heat-exchanging the effluent gas from the fermentor with liquid oxygen produced in the oxygen production unit and of indirectly heat-exchanging the effluent gas with liquid nitrogen produced in the oxygen production unit, thereby separating and removing carbon dioxide from the effluent gas by solidification, and recycling the recovered oxygen to the fermentor.

Preferred embodiments of the apparatus of the invention are described in Claims 2 to 4.

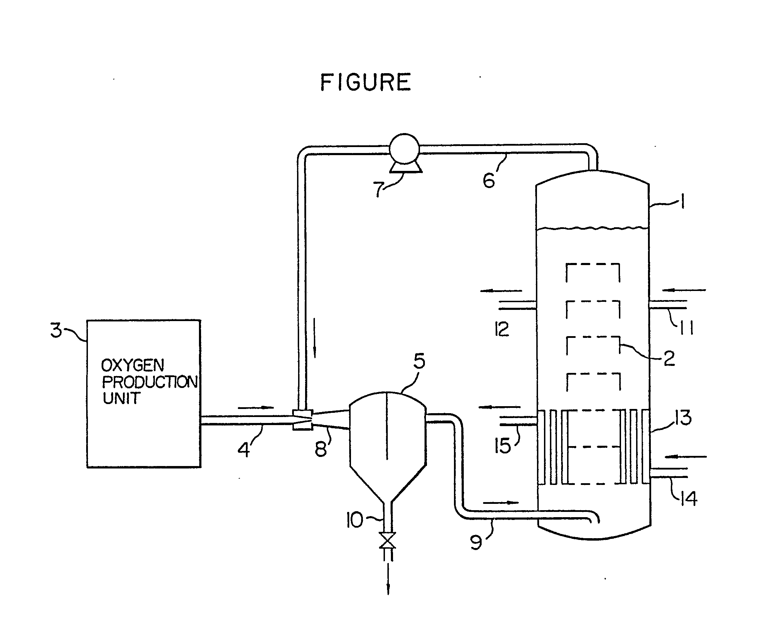

The present invention will be described in detail below, referring to one embodiment shown in the accompanying drawing.

The figure is a schematic flowdiagram showing one embodiment of the present invention.

In Figure, fermentor *1* is provided with cylindrical perforated trays *2* in vertical alignment on different levels to conduct effective gas-liquid contact of an oxygen-enriched gas introduced into the fermentor with a culture medium in the fermentor. Oxygen production unit *3* of cyogenic separation type is provided to supply oxygen necessary for propagation of microorganism cells in fermentor *1*, and is connected to carbon dioxide-removing unit *5* through conduit *4* to lead liquid oxygen produced in the oxygen production unit to the carbon dioxide-removing unit.

On the other hand, effluent gas conduit *6* is provided at the top of fermentor *1* and connected to carbon dioxide-removing unit *5* through recycling blower *7* to lead an effluent gas from the fermentor to the carbon dioxide-removing unit. In carbon dioxide-removing unit *5*, device *8*, for example, an ejector is provided at the inlet and a baffle plate against the ejector at the inside for effectively contacting the liquid oxygen introduced from oxygen production unit *5* through conduit *4* with the effluent gas introduced from the top of fermentor *1* through effluent gas conduit *6* to conduct heat exchange. Carbon dioxide-removing unit *5* is connected to fermentor *2* through conduit *9* to lead the oxygen gas evaporated in the unit and also the oxygen gas recovered from the effluent gas to fermentor *1*. Furthermore, carbon dioxide-removing unit *5* is provided with discharge conduit *10* at the bottom of the unit to discharge solidified carbon dioxide separated and removed from the effluent gas. Furthermore, supply conduit *11* for supplying a culture medium necessary for culturing microorganisms and withdrawal conduit *12* for withdrawing the culture medium are provided at an appropriate level at fermentor *1*. Furthermore, fermentor *1* is provided with cooler *13* for removing the heat generated during the culturing, and supply conduit *14* and discharge conduit *15* for cooling water. In addition, instruments for measuring temperature, pressure, liquid level, pH and dissolved oxygen, and devices and conduits for washing, sterilization, inoculation, acid and alkali supply, and defoaming are provided in the apparatus for culturing microorganisms, though not shown on the drawing.

Flow and function of the present apparatus as shown in Figure will be described below. The liquid oxygen produced in oxygen production unit of cryogenic separation system is supplied to carbon dioxide-removing unit *5* through conduit *4*, and the oxygen gas evaporated in unit *5* is then injected into fermentor *1* at the bottom through conduit *9*. Cylindrical perforated trays *2* are provided in vertical alignment on different levels in fermentor *1*, and the injected oxygen is finely dispersed into the culture medium to undergo effective gas-liquid contact with the culture medium. Most of the oxygen injected into the culture medium is consumed by microorganisms in the culture medium while it goes upwards through the culture medium, and at the same time converted to substantially an equimolar amount of carbon dioxide. The carbon dioxide thus produced arrives at the upper surface of the culture medium charged in fermentor *1*, together with the unconsumed oxygen, and discharged to the outside of fermentor *1* through discharge gas conduit *6*.

In the culturing by aeration with oxygen-enriched gas the production of oxygen-enriched gas is so expensive that it is necessary to increase oxygen utilization to a maximum. Thus, a gas-liquid contactor with high oxygen transfer efficiency must be used for the fermentor and the effluent gas must be recycled.

However, an increase in oxygen utilization in the culturing by aeration with oxygen-enriched gas reduces a flow rate of effluent gas to be discharged to the outside of fermentor *1* and consequently also reduces a rate of carbon dioxide entrained in the effluent gas. Thus, carbon dioxide is inevitably accumulated at a high concentration in the culture medium. The concentration of carbon dioxide accumulated in a culture medium depends upon culturing conditions, for example, kind of strains, concentration of microorganism, concentration of oxygen in aeration gas, aeration rate, etc. Carbon dioxide, when accumulated at a high concentration in a culture medium, gives an adverse effect upon the propagation of microorganisms, and thus the formed carbon dioxide must be removed.

According to the present invention, the effluent gas discharged from fermentor *1* at the top is led to carbon dioxide-removing unit *5* through effluent gas conduit *6* and brought in contact with the liquid oxygen produced in oxygen production unit *3* of cryogenic separation type to separate and remove carbon dioxide from the effluent gas by solidification. The solidifying point of carbon dioxide gas is −56.6°C, and thus carbon dioxide gas can be solidified by cooling it below the solidifying point.

In the production of oxygen at a high rate, an oxygen production unit of cryogenic separation type is used, and can readily produce the necessary amount of oxygen for fermentor *1* and also the necessary amount of a cooling medium for removing the carbon dioxide produced during the culturing, when combined with an aeration fermentor of large scale according to the present invention. Carbon dioxide can be separated and removed from the effluent gas in carbon dioxide-removing unit *5* in this manner, and the recovered oxygen-enriched gas is recycled to fermentor *1* through conduit *9*, together with the oxygen gas evaporated from the liquid oxygen from oxygen production unit *3*. The solidified carbon dioxide separated and removed from the effluent gas in carbon dioxide-removing unit 5 at the same time is discharged to the outside from discharge conduit *10*.

When a cooling rate necessary for solidifying carbon dioxide in the effluent gas cannot be obtained only from the liquid oxygen produced in oxygen production unit *3* of cryogenic separation type, carbon dioxide-reducing unit *10* is indirectly cooled by liquid nitrogen produced in oxygen production unit *3* at the same time. Furthermore, it is also possible to incorporate a cooling device based on compression and adiatatic expansion of effluent gas itself in carbon dioxide-removing unit *5*. Furthermore, it is possible to utilize the coldness of solidified carbon dioxide discharged from carbon dioxide-removing unit 5 to remove the heat generated during the culturing.

The present invention will be described below, referring to Example.

| | |
|---|---|
| Volume of culture medium charged into fermentor: | 1,000 m³ |
| Concentration of microorganism cells: | 100 kg/m³ |
| Oxygen consumption rate per unit weight of culture medium: | 4 moles/kg · cell · hr |
| Respiratory quotient: | 1 mole $CO_2$/mole $O_2$ |
| Oxygen utilization through fermentor: | 60% |
| Culturing temperature: | 30°C |
| Total oxygen consumption rate through fermentor: | $9 \times 10^3$ m³/hr |
| Total oxygen rate to fermentor: | $15 \times 10^3$ m³/hr |
| Total product $CO_2$ rate through fermentor: | $9 \times 10^3$ m³/hr |
| Total effluent gas rate: | $15 \times 10^3$ m³/hr |
| Non-utilized oxygen rate in effluent gas: | $6 \times 10^3$ m³/hr (40%) |

$15 \times 10^3$ m³/hr of the effluent gas discharged from the fermentor was cooled from the culturing temperature of 30°C to about −80°C under one atmosphere to solidify carbon dioxide in the effluent gas. The necessary cooling requirement for solidifying carbon dioxide was about $10^6$ kcal/hr, which could be satisfied by the latent heat of vaporization of liquid oxygen and liquid nitrogen produced in oxygen production unit of cryogenic separation type having a capacity of producing oxygen at the necessary rate for the culturing. That is, the cooling requirement could be satisfied by the latent heat of vaporization of the liquid oxygen having a rate equal to the total oxygen consumption rate through fermentor, i.e. $0.65 \times 10^6$ kcal/hr and the latent heat of vaporization of liquid nitrogen produced at the same time in the oxygen production unit, $2 \times 10^6$ kcal/hr. That is, total coldness obtainable from the liquid oxygen and the liquid nitrogen amounted to about $2.65 \times 10^6$ kcal/hr, and was sufficient for cooling the effluent gas from the fermentor from 30°C to −80°C.

In the present invention, the effluent gas from the fermentor was heat-exchanged directly with liquid oxygen and indirectly with liquid nitrogen efficiently in the carbon dioxide-removing unit of simple structure without any additional energy, except that only a blower was required for supplying the effluent gas from the fermentor to the carbon dioxide-removing unit, as shown in Figure.

The effects of the present invention will be given below in comparison with the conventional apparatuses for aerobically culturing microorganisms by aeration with oxygen-enriched gas.

Example

Microorganisms were cultured in an apparatus as shown in Figure under the following conditions:

Comparative Example 1

Microorganisms were cultured under the same culturing conditions as in Example except that no carbon dioxide-removing unit was used. That is, the effluent gas from the fermentor was discharged to the atmosphere directly.

$6 \times 10^3$ m³/hr of the oxygen gas non-utilized through the fermentor was not recovered in this case, and thus the oxygen gas had to be additionally produced at a rate corresponding to the rate of the unrecovered oxygen.

According to the present invention, the amount of oxygen to be actually supplied to the fermentor can be 40% reduced, as compared with the case of not recovering the non-utilized oxygen gas.

Comparative Example 2

Microorganisms were cultured under the same culturing conditions as in Example, except that the carbon dioxide was removed from the effluent gas from the fermentor by absorption with an aqueous caustic soda solution to recycle the recovered oxygen gas to the fermentor.

$32 \times 10^3$ kg/hr of caustic soda was required for removing $9 \times 10^3$ m³/hr of carbon dioxide formed during the culturing by absorption. In this case, the corresponding amount of water was required for dissolving such a large amount of caustic soda in water. Furthermore, the corresponding amount of the resulting aqueous sodium carbonate solution had to be treated, and when sodium carbonate was recovered as a by-product, an additional concentrating unit was required, making operation complicated.

In this case, use of carbon dioxide was restricted to sodium carbonate, and if there is no need for sodium carbonate at hand, its disposal is a problem.

In the present invention, the necessary oxygen gas for propagation of microorganisms in a fermentor can be supplied from liquid oxygen produced in an oxygen production unit of cryogenic separation type, and at the same time carbon dioxide produced during the culturing can be removed by use of liquid oxygen and liquid nitrogen produced in the oxygen production unit of cryogenic separation type. Accordingly, utilization of oxygen to be introduced into the fermentor can be increased, and the adverse effect of accumulated carbon dioxide upon the propagation of microorganisms can be prevented.

Furthermore, the coldness of solidified carbon dioxide separated and removed in the carbon dioxide-removing unit can be utilized for removal of the heat generated during the culturing. Still furthermore, carbon dioxide in the effluent gas can be separated as pure dry ice, which can be readily used in a wide field of applications.

## Claims

1. An apparatus for culturing microorganisms by aeration, which comprises a fermentor (1) for aerobically culturing microorganisms, an oxygen production unit (3) for producing an oxygen-enriched gas, a carbon dioxide-removing unit (5) for separating and removing carbon dioxide produced during fermentation from an effluent gas from the fermentor (1) and recycling the effluent gas free from the carbon dioxide to the fermentor (1), the fermentor (1) and the carbon dioxide-removing unit (5) being connected to each other in a loop characterised in that the oxygen production unit (3) is of cryogenic separation type and is connected to the carbon dioxide-removing unit (5) at the inlet, in that and the carbon dioxide-recovering unit (5) is of heat exchange type of directly heat-exchanging the effluent gas from the fermentor (1) with liquid oxygen produced in the oxygen production unit (3) and of indirectly heat-exchanging the effluent gas with liquid nitrogen produced in the oxygen production unit (3), thereby separating and removing carbon dioxide from the effluent gas by solidification, and recycling the recovered oxygen to the fermentor (1).

2. An apparatus according to Claim 1, characterized in that the carbon dioxide-removing unit (5) is provided with an ejector (8) at the inlet of mixing the effluent gas with the liquid oxygen, a baffle plate against the ejector inside, and an outlet (10) for discharging solidified carbon dioxide.

3. An apparatus according to Claim 1, characterized in that the fermentor (1) is provided with a plurality of cylindrical perforated trays (2) in vertical alignment at different levels inside and a cooler (13) at the lower part of the fermentor (1), an inlet (9) for the oxygen enriched gas at the bottom, an outlet (12) for the effluent gas at the top, and an inlet (11) and an outlet (15) for culture medium at an appropriate level.

4. An apparatus according to Claim 1, characterized in that, a blower (7) is provided in the loop from the fermentor (1) to the carbon dioxide-removing unit (5).

## Patentansprüche

1. Vorrichtung zur Züchtung von Mikroorganismen durch Belüftung, mit einem Fermentationsbehälter (1) zur aeroben Züchtung von Mikroorganismen, einer Sauerstoff-Produktionseinheit (3) zur Herstellung eines mit Sauerstoff angereicherten Gases, einer Kohlendioxid-Beseitigungseinheit (5) zum Abtrennen und Beseitigen von während der Fermentation erzeugtem Kohlendioxid aus einem vom Fermentationsbehälter (1) strömenden Gas und zum Rückführen des vom Kohlendioxid befreiten abströmenden Gases zum Fermentationsbehälter (1), wobei der Fermentationsbehälter (1) und die Kohlendioxid-Beseitigungseinheit (5) miteinander in einem Kreissystem verbunden sind, dadurch gekennzeichnet, daß die Sauerstoff-Produktionseinheit (3) vom Kryoabscheider-Typ ist und an die Kohlendioxid-Beseitigungseinheit (5) an der Einlaßseite angeschlossen ist, daß die Kohlendioxid-Rückgewinnungseinheit (5) vom Wärmetauschertyp ist, der die Wärme zwischen dem aus dem Fermentationsbehälter (1) abströmenden Gas und dem in der Sauerstoff-Produktionseinheit (3) erzeugten flüssigen Sauerstoff direkt und die Wärme zwischen dem abströmenden Gas und dem in der Sauerstoff-Produktionseinheit (3) erzeugten flüssigen Stickstoff indirekt austauscht, so daß das Kohlendioxid aus dem abströmenden Gas durch Verfestigung abgetrennt und entfernt wird, worauf der wiedergewonnene Sauerstoff zum Fermentationsbehälter (1) rückgeführt wird.

2. Vorrichtung nach Anspruch 1, dadurch gekennzeichnet, daß die Kohlendioxid-Beseitigungseinheit (5) mit einem Ejektor (8) an der Einlaufseite, der das abströmende Gas mit dem flüssigen Sauerstoff vermischt, mit einer Prallplatte gegen die Innenseite des Ejektors, und mit einem Auslaß (10) zum Abführen verfestigten Kohlendioxids versehen ist.

3. Vorrichtung nach Anspruch 1, dadurch gekennzeichnet, daß der Fermentationsbehälter (1) innen mit mehreren zylindrischen perforierten Einsätzen (2) in vertikaler Ausrichtung an verschiedenen Niveaus und einem Kühler (13) im unteren Teil des Fermentationsbehälters (1), einem Einlaß (9) für das mit Sauerstoff angereicherte Gas am Boden, einem Auslaß (12) für das abströmende Gas an der Oberseite, und mit einem Einlaß (11) und einem Auslaß (15) für das Kulturmedium an einem geeigneten Niveau versehen ist.

4. Vorrichtung nach Anspruch 1, dadurch gekennzeichnet, daß ein Gebläse (7) im Kreislauf vom Fermentationsbehälter (1) zur Kohlendioxid-Beseitigungseinheit (5) vorgesehen ist.

## Revendications

1. Appareil pour cultiver les microorganismes par aération, qui comprend un fermenteur (1) pour cultiver des microorganismes en aérobei, une installation de production d'oxygène (3) pour produire un gaz enrichi en oxygène, une installation d'élimination du gaz carbonique (5) pour séparer et éliminer le gaz carbonique produit au cours de la fermentation d'un gaz se dégageant du fermenteur (1) et recycler le gaz se dégageant exempt de gaz carbonique dans le fermenteur (1), le fementeur (1) est l'installation d'élimination du gaz carbonique (5) étant reliés l'un à l'autre dans une boucle, caractérisé par le fait que l'installation de production d'oxygène (3) est du type à séparation cryogénique et est reliée à l'installation d'élimination de gaz carbonique (5) à l'entrée, et par le fait que l'installation de récupération du gaz carbonique (5) est du type à échange de chaleur échangeant directement de la chaleur entre le gaz se dégageant du fermenteur (1) et l'oxygène liquide produit dans l'installation de production d'oxygène (3) et échangeant indirectement de la chaleur entre le gaz se dégageant et de l'azote liquide produit dans l'installation de production d'oxygène (3), séparant et éliminant ainsi du gaz carbonique du gaz se dégageant par solification, et recylant l'oxygène récupéré dans le fermenteur (1).

2. Appareil selon la revendication 1, caractérisé par le fait que l'installation d'élimination du gaz carbonique (5) est munie d'un éjecteur (8) à l'entrée pour mélanger le gaz se dégageant avec l'oxygène liquide, d'une plaque déflectrice contre l'intérieur de l'éjecteur, et d'une sortie (10) pour évacuer le gaz carbonique solidifié.

3. Appareil selon la revendication 1 caractérisé par le fait que le fementeur (1) est muni d'une pluralité de plateaux cylindriques perforés (2) en alignement vertical à différents niveaux à l'intérieur et d'un réfrigérant (13) à la partie inférieure du fermenteur (1), d'une entrée (9) pour le gaz enrichi en oxygène à la base, d'un orifice de sortie (12) pour le gaz se dégageant au sommet, et d'une entrée (11) et d'une sortie (12) pour le milieu de culture à un niveau approprié.

4. Appareil selon la revendication 1, caractérisé par le fait qu'un ventilateur (7) est prévu dans la boucle allant du fermenteur (1) à l'installation d'élimination du gaz carbonique (5).

FIGURE

OXYGEN
PRODUCTION
UNIT